# EUROPEAN PATENT SPECIFICATION

(11) **EP 3 598 233 B1**
(45) Date of publication and mention of the grant of the patent: **11.11.2020**
(21) Application number: 19165282.5
(22) Date of filing: 26.03.2019
(51) Int. Cl.: G03F 7/031, C08F 2/48

(54) **LIQUID PHOTOINITIATING COMPOUND, METHOD OF INITIATING A POLYMERIZATION REACTION AND PHOTOPOLYMERIZABLE COMPOSITION**
FLÜSSIGER FOTOINITIATOR, VERFAHREN ZUR INITIIERUNG EINER POLYMERISATIONSREAKTION UND PHOTOPOLYMERISIERBARE ZUSAMMENSETZUNG
PHOTOINITIATEUR LIQUIDE , MÉTHODE D'INITIATION DE RÉACTION DE POLYMÉRISATION ET COMPOSITION PHOTOPOLYMÉRISABLE

(30) Priority: 17.07.2018 TW 107124654
(43) Date of publication of application: 22.01.2020
(73) Proprietor: Chitec Technology Co., Ltd., Taipei City 106 (TW)
(72) Inventor: Chang, Wei-Chun, 106 TAIPEI CITY (TW); Chiu, Chingfan Chris, 106 TAIPEI CITY (TW); Wu, Huang-Min, 106 TAIPEI CITY (TW)
(74) Representative: dompatent von Kreisler Selting Werner - Partnerschaft von Patent- und Rechtsanwälten mbB

(56) References cited:
- EP-A1- 1 410 109
- EP-A1- 2 509 948
- EP-A1- 2 664 629
- JP-A- H04 164 901

## Description

### CLAIM FOR PRIORITY

### BACKGROUND OF THE INVENTION

### Field of the Invention

The present invention relates to a liquid photo initiating compound and photopolymerizable compositions using the same.

### Descriptions of the Related Art

A photoinitiator is a substance that forms free radicals via Norrish Type I photo-fragmentation after absorbing the energy of visible or UV light, which can initiate polymerization of monomers or oligomers to provide a curing effect.

In 1990, Ciba Specialty Corporation introduced Irgacure® 369, which is the first photoinitiator specifically designed for dark color UV ink (related patent: US 5,077,402). Irgacure® 369 is in solid form and its structure is shown below.

### [Irgacure® 369]

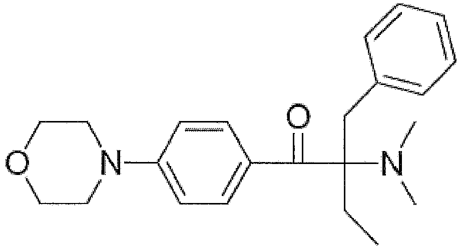

Irgacure® 369 has an outstanding photo speed and other advantages like odorless prior to and after curing. However, it suffers from poor solubility in most UV inks and thus requires additional grinding and heating processes to facilitate dissolution. Even though, Irgacure® 369 is prone to precipitate when dosage is over 4 wt% and when handled in cold areas. Irgacure® 369 has recently been shown to have reproductive toxicity and thus is under radar in EU to be prohibited from use in food contact applications.

In order to improve the solubility issue, Ciba Specialty Corporation introduced another solid photoinitiator product, i.e., Irgacure® 379 (related patent: US 7,247,659 B2). Irgacure® 379 does have improved solubility in some monomers and resins than Irgacure® 369. Still, it is not easy to use in its solid form that requires grinding and heating processes in order to dissolve into an ink composition. Furthermore, Irgacure® 379 also has reproductive toxicity.

### [Irgacure® 379]

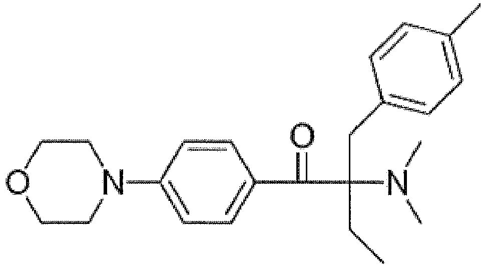

In recent years, the demand for low viscosity UV inks such as digital UV and flexo UV inks has increased significantly. A photoinitiator in liquid form with high solubility is therefore highly desired to the low viscosity ink as these inks do not provide sufficient shearing force to perform the grinding and dispersion processes for solid photoinitiators.

Examples of liquid photoinitiators include the following compounds 93, 95, 96 and 100 as disclosed in US 5,077,402. But these compounds are not commercialized because their poor photospeeds as well as odor issues.

### [US 5,077,402]

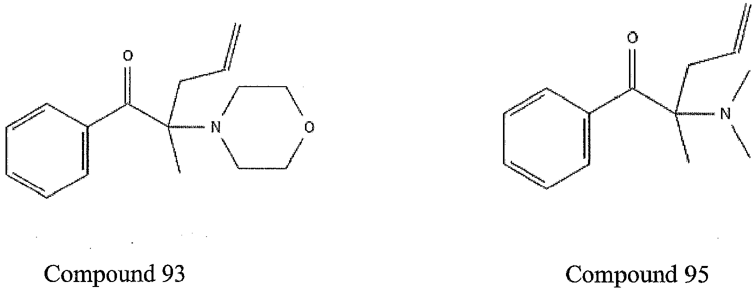

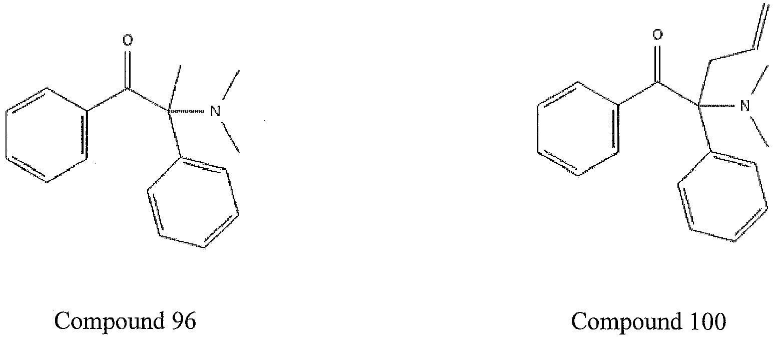

TW 1277834 discloses a liquid photoinitiator in Example 3. Even though the liquid photoinitiator is odorless, it is not commercialized due to its poor photo speed which is about one tenth that of Irgacure® 369.

### [Example 3 of TW 1277834]

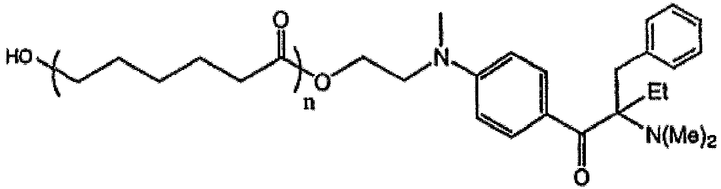

The following compound I is also a liquid photoinitiator and has a photo speed comparable to that of Irgacure® 369. Unfortunately, the compound I has a serious odor issue after curing.

### [Compound I]

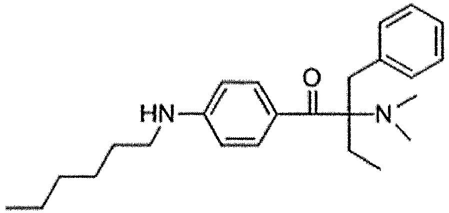

TW 1564276 also discloses a liquid photoinitiator, i.e., the following compound IIa, which is comparable to Irgacure® 369 in terms of photo speed and is odorless prior to or after curing. However, the compound IIa has high acute oral toxicity. Besides, it is in dark-brown color which is not satisfactory for some colors.

### [Compound IIa of TW 1564276]

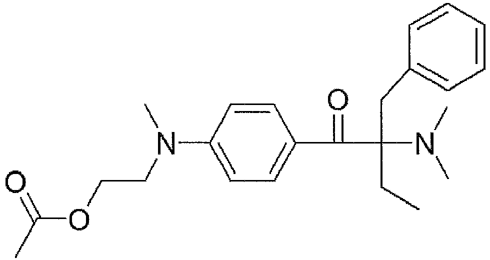

Therefore, conventional liquid photoinitiators with individual defects for dark and black inks are far from satisfactory.

EP 2509948 A1 discloses a photoinitiator of Formula: 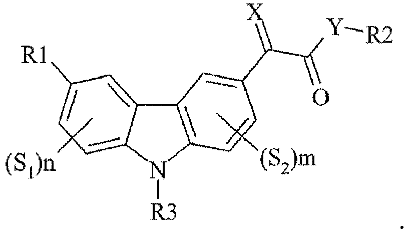 JP H04 164901 A discloses a photoinitiator of Formula: 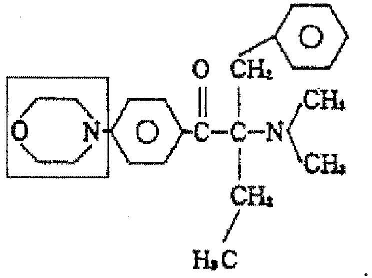 EP 1 410 109 A1 discloses a photoinitiator of Formula: 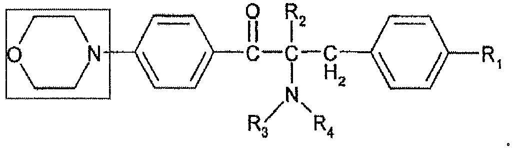 EP 2 664 629 A1 discloses a photoinitiator of Formula:

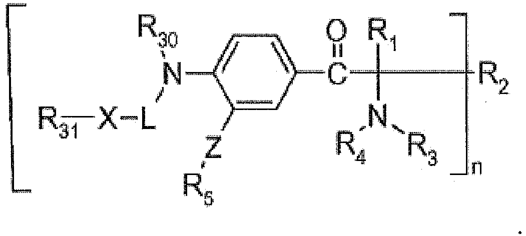

### SUMMARY OF THE INVENTION

In view of the unsatisfaction of conventional photoinitiators, the present invention provides a photoinitiator derived from oxazolidine, which improves all the aforementioned defects associated conventional liquid photoinitiators for the black ink system as: (1) it becomes a liquid state at 50°C; (2) it has a photo speed comparable to that of Irgacure® 369; (3) it is odorless prior to and after curing; and (4) it does not have acute oral toxicity. The embodiments of the present invention are reflected in independent claims 1, 4 and 5. The preferred embodiments of the present invention are reflected in dependent claims 2, 3, and 6 to 11. Furthermore, its light color and high purity perfectly meet the requirements of the industry regarding liquid photoinitiators. Accordingly, the present invention involves at least the objectives described below.

It should be noted that neither US 5,077,402 nor US 7,732,504 B2 discloses the photoinitiator of the present invention, even though the photoinitiators disclosed in these patents may have an oxazolidine structure. Furthermore, persons having ordinary skill in the art cannot expect the advantages of the photoinitiator of the present invention based on the general teaching of US 5,077,402 or US 7,732,504 B2. The advantages include being liquid at a low temperature of 50°C, high solubility in various monomers and oligomers, odorless, comparable photo speed to Irgacure 369, and no acute oral toxicity.

An objective of the present invention is to provide a liquid photo initiating compound, which is represented by the following Formula I: 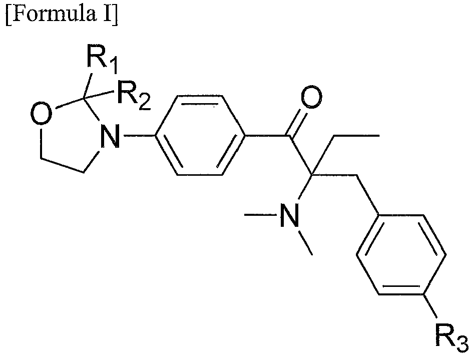 wherein, in Formula I, R₁ and R₂ are independently H or C₁-C₃ alkyl, and R₃ is H or methyl. Examples of C₁-C₃ alkyl include methyl, ethyl, n-propyl and isopropyl.

In some embodiments of the present invention, the aforementioned compound is represented by the following Formula la:

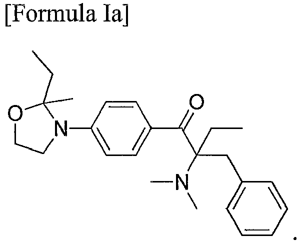

Another objective of the present invention is to provide a method of initiating a polymerization reaction by using the aforementioned liquid photo initiating compound.

Yet another objective of the present invention is to provide a photopolymerizable composition, comprising:
a first photoinitiator, which is the aforementioned liquid photo initiating compound;
a photopolymerizable component; and
an optional solvent.

In some embodiments of the present invention, the photopolymerizable component is an olefinic unsaturated monomer, an olefinic unsaturated oligomer, or a combination thereof. Examples of the olefinic unsaturated monomer include acrylate-based monomers. Examples of the olefinic unsaturated oligomer include acrylate-based oligomers.

In some embodiments of the present invention, the photopolymerizable composition further comprises a second photoinitiator selected from the group consisting of acylphosphine oxides, 9,10-dialkyloxyanthracene ( 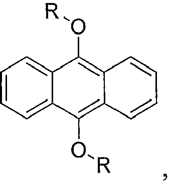 wherein R is alkyl), and combinations thereof.

In some embodiments of the present invention, the photopolymerizable composition further comprises a photosensitizer selected from the group consisting of benzophenones, thioxanthones, Michler's ketones, anthraquinone, and combinations thereof.

In some embodiments of the present invention, the photopolymerizable composition further comprises an additive selected from the group consisting of pigments, amine synergists, and combinations thereof.

In some embodiments of the present invention, the content of the first photoinitiator ranges from 0.1 wt% to 15 wt% based on the total weight of the photopolymerizable composition.

To render the above objectives, technical features and advantages of the present invention more apparent, the present invention will be described in detail with reference to some embodiments hereinafter.

### BRIEF DESCRIPTION OF THE DRAWINGS

Not applicable.

### DESCRIPTION OF THE PREFERRED EMBODIMENT

Hereinafter, some specific embodiments of the present invention will be described in detail. The present invention may be embodied in various embodiments and should not be limited to the specific embodiments described in the specification.

Unless it is additionally explained, the expressions "a,", "an", "the," or the like recited in the specification (especially in the claims) should include both the singular and the plural forms.

Unless it is additionally explained, the term such as "first", "second" or the like is used to distinguish different elements or components, not terms supplying a numerical limit.

Unless it is additionally explained, the term "alkyl" recited in the specification (especially in the claims) includes linear, branched and/or cyclic alkyl groups.

### Liquid photo initiating compound

The liquid photo initiating compound of the present invention is represented by the following Formula I:

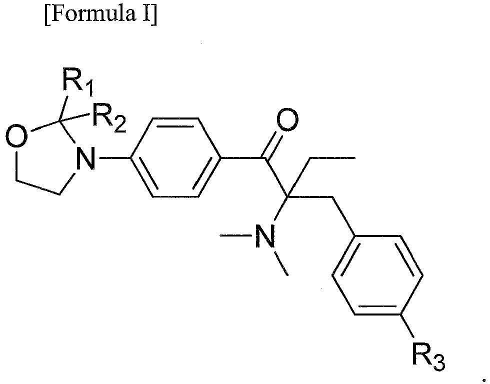

In formula I, R₁ and R₂ are independently H or C₁-C₃ alkyl, and R₃ is H or methyl. In some embodiments of the present invention, R₁ and R₂ are independently methyl, ethyl, n-propyl or isopropyl, and R₃ is H. In the appended examples, the first photoinitiator is represented by the following Formula la:

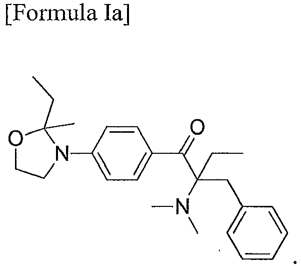

The synthesis of the liquid photo initiating compound of the present invention will be described in the appended examples.

### Uses of liquid photo initiating compound

The liquid photo initiating compound of the present invention is liquid at a low temperature of 50°C. Therefore, it can be used as a liquid photoinitiator to trigger polymerization and crosslinking of photopolymerizable monomers or oligomers and thus, provide a curing effect. The liquid photo initiating compound of the present invention surprisingly shows properties quite different from conventional photoinitiators and has many advantages. Specifically, the liquid photo initiating compound of the present invention has excellent photo speed performance, excellent solubility and light color and is in liquid state at a low temperature of 50°C. When used in a photopolymerizable system, the influence of the liquid photo initiating compound of the present invention on viscosity is very minor. The liquid photo initiating compound of the present invention also has long storage stability. Therefore, the liquid photo initiating compound of the present invention is particularly useful for low viscosity UV inks (e.g., an ink with a viscosity of less than 1000 cP), light color UV inks, color resists, black matrixes and solder masks. Furthermore, the liquid photo initiating compound of the invention has high purity that can meet the chemical substance registration regulations of nations of the world.

Accordingly, the present invention also provides a photopolymerizable composition, comprising a first photoinitiator, a photopolymerizable component and an optional solvent, wherein the first photoinitiator is the compound represented by Formula I.

The species of the photopolymerizable component is not particularly limited and can be any substance that is photopolymerizable in the presence of a photoinitiator, including the substance that is generally used in photopolymerizable systems, like UV inks. In some embodiments of the present invention, the photopolymerizable component is selected from the group consisting of olefinic unsaturated monomers, olefinic unsaturated oligomers and combinations thereof. Examples of the olefinic unsaturated monomer include but are not limited to acrylate-based monomers, and examples of the olefinic unsaturated oligomer include but are not limited to acrylate-based oligomers.

The solvent is optional and can be any solvent that dissolves or disperses the components of the composition but does not react with the components, including those generally used in photopolymerizable systems such as UV inks. Examples of the solvent include but are not limited to water; aliphatic hydrocarbons, such as dichloromethane, trichloromethane, tetrachloromethane, n-hexane, and cyclohexane; aromatic hydrocarbons, such as toluene, benzene, and xylene; ketones, such as acetone, methyl ethyl ketone, isobutyl ketone and cyclohexanone; esters, such as ethyl acetate and butyl acetate; alcohols, such as methanol, ethanol, n-propanol and isopropanol; and ethers, such as dimethylether, diethylether, and methylethylether.

In the photopolymerizable composition of the present invention, the amount of the first photoinitiator is not particularly limited and can be optionally adjusted by persons having ordinary skill in the art. To obtain a better photocuring efficacy, the amount of the photoinitiator is usually from 0.1 wt% to 15 wt%, such as 1 wt%, 2 wt%, 3 wt%, 4 wt%, 5 wt%, 6 wt%, 7 wt%, 8 wt%, or 9 wt%, based on the total weight of the photopolymerizable composition. However, the present invention is not limited thereto.

In addition to the first photoinitiator, the photopolymerizable composition of the present invention can optionally further include other components that are advantageous to the efficacy of photoinitiation, like photosensitizing components or other photo initiating components. Therefore, in some embodiments of the present invention, the photopolymerizable composition further comprises a second photoinitiator, a photosensitizer, or a combination thereof.

The second photoinitiator can be any photo initiating component other than the first photoinitiator. Examples of the second photoinitiator include but are not limited to acylphosphine oxides, 9,10-dialkyloxyanthracene, and combinations thereof. The species of the photosensitizer is not particularly limited. Examples of the photosensitizer include but are not limited to benzophenones, thioxanthones, Michler's ketones, anthraquinone, and combinations thereof. In the appended examples, 2,4-diethylthioxanthone (product name: Chivacure® DETX) is used as a second photosensitizer in the photopolymerizable composition. The amount of the second photoinitiator or the photosensitizer is not particularly limited and can be optionally adjusted by persons having ordinary skill in the art depending on the need.

The photopolymerizable composition of the present invention may optionally further comprise one or more additives to impart desired properties to the cured product of the composition. For example, a pigment can be added to provide color to the cured product, and an amine synergist can be added to improve curing performance, for example, to improve the surface curing speed of composition and to enhance the mirror surface effect after curing. Examples of the pigment include titanium oxide, carbon black, cadmium red, molybdate red, chrome yellow, cadmium yellow, titanium yellow, chromium oxide, cobalt titanate green, cerulean blue, copper blue, azo pigments, phthalocyanine pigments, quinacridone based pigments, isoindolinone based pigments, perylene based pigments, thio indigo based pigments, and metal complex pigments. As used herein, an amine synergist is well-known to persons having ordinary skill in the art and refers to a reactive amine that can promote the photoinitiation, such as a low-molecular weight tertiary amine. Examples of the amine synergist include but are not limited to methyldiethanolamine, dibutylethanolamine, triethylamine and triethanolamine. Commercially available products of the amine synergist include Chivacure® 115, Chivacure® EPD, Chivacure® OPD, etc. available from Chitec Technology Co., Ltd.

The present invention is further illustrated by the following embodiments.

### Examples

### Example 1: preparation of liquid photo initiating compound represented by Formula Ia

34 g 2-(dimethylamino)-1-[4-[(2-hydroxyethyl)amino]phenyl]-2-(phenylmethyl)-1-butanone (CAS 862589-48-8); the synthesis method of it may refer to TW 1277834), 13.0 g 2,2-dimethoxybutane (CAS 3453-99-4), 0.17 g PTSA (p-toluenesulfonic acid), and 100 mL toluene were added in sequence to a 250 mL three-necked flask at room temperature and then the reaction as shown below was carried out at reflux. The by-product methanol produced by this reaction was removed by Dean-Stark evaporator.

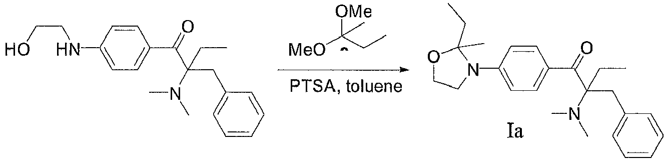

The reaction was monitored by High Performance Liquid Chromatography (HPLC). After the reaction was completed, the reaction product was cooled to room temperature and washed with 17 g pure water for three times. The organic layer was collected and then concentrated under vacuum to obtain the compound represented by Formula Ia (hereinafter "photo initiating compound Ia") as a yellowish and viscous liquid. The yield is 95%.

The photo initiating compound Ia was subjected to nuclear magnetic resonance analysis, and the results are as follows:
Nuclear magnetic ¹H NMR(500 MHz, CDCl₃): 0.70 (t, J = 7.5 Hz, 3H), 0.82 (t, J = 7.5
resonance analysis: Hz, 3H), 1.85-1.89 (m, 1H), 1.94-1.99 (m, 1H), 2.04-2.09 (m, 1H), 2.13-2.18(m, 1H), 2.36 (s, 6H),3.16-3.23 (m, 2H), 3.18 (d, J = 14.0Hz, 1H), 3.22 (d, J =14.0Hz, 1H), 4.06-4.10 (m, 1H), 4.12-4.26 (m, 1H), 6.60 (d, J = 9.0 Hz, 2H), 7.16-7.28 (m, 6H), 8.34 (d, J = 9.0 Hz, 2H)
   ¹³C NMR(500 MHz, CDCl₃): 7.7, 9.8, 23.8, 27.8, 31.0, 35.5, 39.1, 48.4, 63.2, 73.8, 96.2, 111.5, 125.8, 125.9, 127.9, 131.3, 132.2, 139.6, 147.1,201.6

### Example 2: Preparation of a liquid photo initiating compound represented by Formula Ib

34 g 2-(dimethylamino)-1-[4-[(2-hydroxyethyl) amino]phenyl]-2-(phenylmethyl)-1-butanone, 12.0 g 2,2-dimethoxypropane (CAS 77-76-9), 0.17 g PTSA (p-toluenesulfonic acid), and 100 mL toluene were added to a 250 mL three-necked flask at room temperature in sequence and the reaction as shown below was carried out at a condition the same as the previous example.

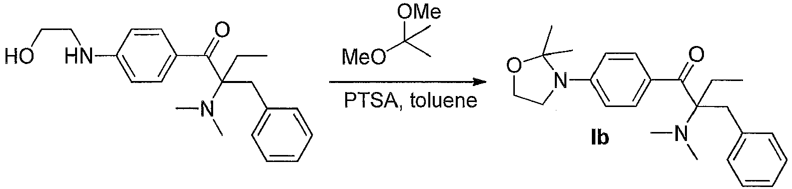

The reaction was monitored by High Performance Liquid Chromatography (HPLC). After the reaction was completed, the reaction product was extracted with 17 g pure water for three times. The organic layer was collected and then concentrated under vacuum to obtain the compound represented by Formula Ib (hereinafter referred to as "photo initiating compound Ib") as a yellowish and viscous liquid. The yield is 93%.

The photo initiating compound Ib was subjected to nuclear magnetic resonance analysis, and the results are as follows:

| | |
|---|---|
| Nuclear magnetic resonance analysis: | ¹H NMR(500 MHz, CDCl₃): 0.69 (t, J = 7.5Hz, 3H), 1.64 (s, 6H), 1.83- 1.90 (m, 1H), 2.02-2.17 (m, 1H), 2.36 (s, 6H), 3.17 (d, J = 14.0Hz, 1H), 3.22 (d, J = 14.0Hz, 1H), 3.53 (t, J = 6.5Hz, 2H), 4.11 (t, J = 6.5Hz, 2H), 6.60 (d, J = 9.0Hz, 2H), 7.15-7.28(m, 6H), 8.35(d, J = 9.0Hz, 2H) |
| | ¹³C NMR(500 MHz, CDCl₃): 9.8, 25.4, 27.8, 35.5, 39.1, 47.8, 62.9, 73.9, 93.9, 111.8, 125.8, 125.9, 127.9, 131.3, 132.2, 139.6, 147.0, 201.6 |

### Example 3: Solubility test

Mixtures I to III were prepared according to the composition provided below, and then 6 parts by weight of photoinitiator Irgacure® 369 (available from IGM), photoinitiator R-gen® 919 (available from Chitec Technology Co., Ltd.) or photo initiating compound Ia was added to each of the mixtures I to III. The obtained mixtures were ultrasonicated for 1 hour at room temperature, and the dissolution of each of Irgacure® 369, R-gen® 919 and photo initiating compound Ia was observed and described in the following Table 1.

| | |
|---|---|
| Mixture I: | a mixture of 50 parts by weight of 1,6-hexanediol diacrylate (product name: EM221, Eternal Materials Co., Ltd.) and 50 parts by weight of polyester tetraacrylate (product name: Oligomer 6325-100, Eternal Materials Co., Ltd.) with a viscosity of 41.2 cP |
| Mixture II: | a mixture of 50 parts by weight of trimethylolpropane triacrylate (product name: EM231, Eternal Materials Co., Ltd.) and 50 parts by weight of polyester tetraacrylate (product name: Oligomer 6325-100, Eternal Materials Co., Ltd.) with a viscosity of 349.5 cP |
| Mixture III: | a mixture of 50 parts by weight of ethoxylated pentaerythritol tetraacrylate (product name: EM2411, Eternal Materials Co., Ltd.) and 50 parts by weight of polyester tetraacrylate (product name: Oligomer 6325-100, Eternal Materials Co., Ltd.) with a viscosity of 433.0 cP |

**Table 1: Dissolution of Irgacure® 369, R-gen® 919 and photo initiating compound Ia after one-hour ultrasonication at room temperature**

| Photoinitiator | Mixture I | Mixture II | Mixture III |
|---|---|---|---|
| Irgacure® 369 | Completely dissolved | Only a small part was dissolved | Only a small part was dissolved |
| R-gen® 919 | Completely dissolved | Completely dissolved | Completely dissolved |
| Photo initiating compound Ia | Completely dissolved | Completely dissolved | Completely dissolved |

As shown in Table 1, the photo initiating compound Ia completely dissolved in each of the three mixtures after one-hour ultrasonication at room temperature. The results indicate that the photo initiating compound Ia is much better than the solid photoinitiator Irgacure® 369 and comparable to R-gen® 919 in terms of solubility.

### Example 4: Viscosity variation test

4 parts by weight of photoinitiators Irgacure® 369, R-gen® 919, and photo initiating compound Ia were individually added to each of the mixtures I to III, and the obtained mixtures were exposed to a 0°C atmosphere for 7 days. The viscosities before and after the exposure were measured and tabulated in the following Table 2.

**Table 2: Viscosities (cP) before and after exposure to 0°C atmosphere for 7 days**

| Photoinitiator | Mixture I | | Mixture II | | Mixture III | |
|---|---|---|---|---|---|---|
| | Initial | 7 days | Initial | 7 days | Initial | 7 days |
| None | 41.2 | 50.1 | 349.5 | 401.0 | 433.0 | 548.0 |
| Irgacure® 369 | 52.7 | 60.4 | 385.0 | Precipitate | 487.0 | Precipitate |
| R-gen® 919 | 42.2 | 51.0 | 354.0 | 408.0 | 445.5 | 544.0 |
| Photo initiating compound Ia | 41.6 | 51.2 | 351.3 | 406.7 | 433.1 | 547.8 |

As shown in Table 2, the photo initiating compound IA has the lowest influence on the viscosity of the mixture. In addition, after the exposure to a 0°C atmosphere for 7 days, the viscosity variation of the mixture with the photo initiating compound Ia is similar to that of the mixture without photoinitiator, and no precipitate was observed in the mixture with the photo initiating compound Ia. The results indicate that the photo initiating compound Ia is better than Irgacure® 369 in terms of solubility and resin compatibility and therefore is very useful.

### Example 5: Photo speed performance test

The black flexo formulation containing Oligomer 6325-100, EM2411 and carbon black (viscosity: 1750 cP) was mixed with a photoinitiator to carry out the photo speed performance test. Specifically, 100 parts by weight of the black flexo formulation and 2 parts by weight of Chivacure® DETX (available from Chitec Technology Co., Ltd.) were mixed, and then 6 parts by weight of Irgacure® 369, R-gen® 998 (available from Chitec Technology Co., Ltd.), R-gen® 919, or photo initiating compound Ia was added to the obtained mixture. The resultant mixture was coated on a printable pearlescent film (wet film thickness: 25.15 µm); and the coated printable pearlescent film was exposed to an UV light (Fusion F300, D-bulb, 300 W/inch) for curing. The photo speed performance was observed and tabulated in the following Table 3.

**Table 3: Photo speed performance of photoinitiator**

| Photoinitiator | Irgacure® 369 | R-gen® 998 | R-gen® 919 | Photo initiating compound Ia |
|---|---|---|---|---|
| Photo speed (m/minute) | 120 | 70 | 120 | 120 |

Theoretically, photoinitiators with electron withdrawing groups, such as the photo initiating compound la, usually have a lower photo speed due to the blue shift effect. However, as shown in Table 3, the photo initiating compound Ia surprisingly has an outstanding photo speed which is comparable to those of Irgacure® 369 and R-gen® 919 and significantly better than that of R-gen® 998.

### Example 6: Photoinitiator dosage test

The commercially available black flexo ink without a photoinitiator was mixed with a photoinitiator to carry out the photo speed performance test, and the effect of the amount of photo initiating compound Ia on the photo speed performance was evaluated. Specifically, 100 parts by weight of the black flexo formulation and 2 parts by weight of Chivacure® DETX (photoinitiator; Chitec Technology Co., Ltd.) were mixed, and then a specific amount of Irgacure® 369 or photo initiating compound Ia as shown in Table 4 was added to the obtained mixture. The resultant mixture was coated on a PET film with primer (means for improving adhesion) (dry film thickness: 5 µm). The coated PET film was exposed to an UV light (wavelength: 365 nm; 4 W/inch) for curing. The photo speed performance in each case was observed and tabulated in the following Table 4.

**Table 4: Photo speed performance of photoinitiator in different amounts**

| | Amounts (parts by weight) | Curing performance (times of UV light treatment) | | | | |
|---|---|---|---|---|---|---|
| | | 1 | 2 | 3 | 4 | 5 |
| Irgacure® 369 | 4 | X | X | X | Δ | ○ |
| | 8 | Incomplete dissolution | | | | |
| Photo initiating compound Ia | 4 | X | X | X | Δ | ○ |
| | 8 | Δ | ○ | | | |
| | 12 | ○ | | | | |

### Description for curing results:

| | |
|---|---|
| X | Not tack-free |
| Δ | Tack-free but not through-cure |
| ○ | Tack-free and through-cure |

As shown in Table 4, the tested black flexo ink is an ink system that is difficult to be photocured, and the photocuring efficiency can be improved by increasing the amount of the photoinitiator. However, when 8 parts by weight of Irgacure® 369 was added, like the result of the solubility test, the photoinitiator could not be completely dissolved in the ink system, making it impossible to carry out a photo speed test. By contrast, when the amount of the photo initiating compound Ia was increased, no solubility issue was observed even if the amount reaches 12 parts by weight. Furthermore, increasing the amount of the photo initiating compound Ia from 4 parts by weight to 8 parts by weight can significantly improve the photocuring speed; and only one time of UV light treatment was required to achieve sufficient curing when the amount of the photo initiating compound Ia was 12 parts by weight.

### Example 7: Acute oral toxicity test

The median lethal dose (LD50) test was carried out for each of Irgacure® 369, R-gen® 998, R-gen® 919 and photo initiating compound Ia in accordance with Test Guidelines 423: acute oral toxicity, Organization for Economic Co-operation and Development (OECD). The results are tabulated in the following Table 5.

**Table 5: Median lethal dose (LD50) of photoinitiator**

| Photo initiator | Irgacure® 369 | R-gen® 998 | R-gen® 919 | Photo initiating compound Ia |
|---|---|---|---|---|
| LD50 (mg/kg) | > 5000 | 2500 | 500 | > 5000 |

As shown in Table 5, the median lethal dose (LD50) of the photo initiating compound Ia is comparable with that of Irgacure® 369, both exceeding 5000 mg/kg, which is considered as no acute oral toxicity.

As can be seen from the results of Examples 1 to 7, the liquid photo initiating compound of the present invention has excellent photo speed performance, excellent solubility, light color and no acute oral toxicity. The liquid photo initiating compound of the present invention is in liquid state at a low temperature of 50°C. When being used in a photopolymerizable system, the influence of the liquid photo initiating compound of the present invention on viscosity is very minor. The liquid photo initiating compound of the present invention also has long storage stability. Therefore, the liquid photo initiating compound of the present invention is particularly useful for low viscosity UV inks, light color UV inks, color resists, black matrixes and solder masks.

The above examples are used to illustrate the principle and efficacy of the present invention and show the inventive features thereof. Therefore, the scope of protection of the present invention is that as defined in the claims as appended.

## Claims

1. A liquid photo initiating compound, which is represented by the following Formula I: 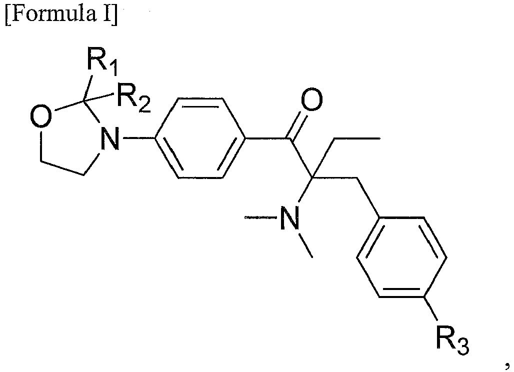 wherein, in Formula I, R₁ and R₂ are independently H or C₁-C₃ alkyl, and R₃ is H or methyl.

2. The compound of Claim 1, wherein R₁ and R₂ are independently methyl, ethyl, n-propyl or isopropyl.

3. The compound of Claim 2, which is represented by the following Formula la:

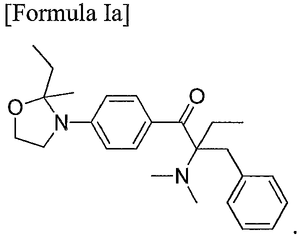

4. A method of initiating a polymerization reaction by using the liquid photo initiating compound of any one of Claims 1 to 3.

5. A photopolymerizable composition, comprising:
a first photoinitiator, which is the liquid photo initiating compound of any one of Claims 1 to 3;
a photopolymerizable component; and
an optional solvent.

6. The photopolymerizable composition of Claim 5, wherein the photopolymerizable component is an olefinic unsaturated monomer, an olefinic unsaturated oligomer, or a combination thereof.

7. The photopolymerizable composition of Claim 6, wherein the photopolymerizable component is an acrylate-based monomer, an acrylate-based oligomer, or a combination thereof.

8. The photopolymerizable composition of any one of Claims 5 to 7, further comprising a second photoinitiator selected from the group consisting of acylphosphine oxides, 9,10-dialkyloxyanthracene ( 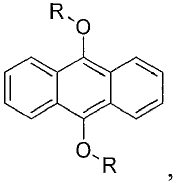 wherein R is alkyl), and combinations thereof.

9. The photopolymerizable composition of any one of Claims 5 to 8, further comprising a photosensitizer selected from the group consisting of benzophenones, thioxanthones, Michler's ketones, anthraquinone, and combinations thereof.

10. The photopolymerizable composition of any one of Claims 5 to 9, further comprising an additive selected from the group consisting of pigments, amine synergists, and combinations thereof.

11. The photopolymerizable composition of any one of Claims 5 to 10, wherein the content of the first photoinitiator ranges from 0.1 wt% to 15 wt% based on the total weight of the photopolymerizable composition.

## Patentansprüche

1. Flüssige Photoinitiatorverbindung, die durch die folgende Formel I dargestellt wird: 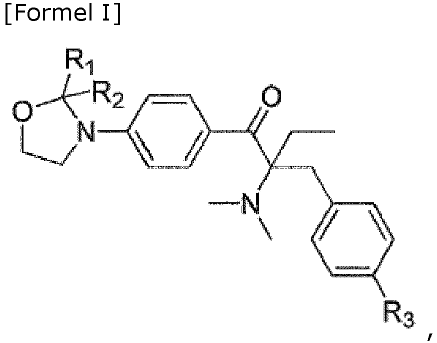 wobei in Formel I R₁ und R₂ unabhängig H oder C₁-C₃-Alkyl sind und R₃ = H oder Methyl ist.

2. Verbindung gemäß Anspruch 1, wobei R₁ und R₂ unabhängig Methyl, Ethyl, n-Propyl oder Isopropyl sind.

3. Verbindung gemäß Anspruch 2, die durch die folgende Formel Ia dargestellt wird:

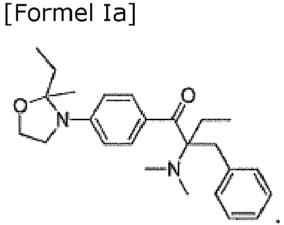

4. Verfahren zum Einleiten einer Polymerisationsreaktion durch Verwendung der flüssigen Photoinitiatorverbindung gemäß einem der Ansprüche 1 bis 3.

5. Photopolymerisierbare Zusammensetzung, umfassend:
einen ersten Photoinitiator, bei dem es sich um die flüssige Photoinitiatorverbindung gemäß einem der Ansprüche 1 bis 3 handelt;
eine photopolymerisierbare Komponente; und
ein optionales Lösungsmittel.

6. Photopolymerisierbare Zusammensetzung gemäß Anspruch 5, wobei die photopolymerisierbare Komponente ein olefinisch ungesättigtes Monomer, ein olefinisch ungesättigtes Oligomer oder eine Kombination davon ist.

7. Photopolymerisierbare Zusammensetzung gemäß Anspruch 6, wobei die photopolymerisierbare Komponente ein Monomer auf Acrylatbasis, ein Oligomer auf Acrylatbasis oder eine Kombination davon ist.

8. Photopolymerisierbare Zusammensetzung gemäß einem der Ansprüche 5 bis 7, weiterhin umfassend einen zweiten Photoinitiator, der aus der Gruppe ausgewählt ist, die aus Acylphosphinoxiden, 9,10-Dialkyloxyanthracen 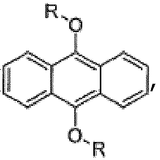 wobei R Alkyl ist) und Kombinationen davon besteht.

9. Photopolymerisierbare Zusammensetzung gemäß einem der Ansprüche 5 bis 8, weiterhin umfassend einen Photosensibilisator, der aus der Gruppe ausgewählt ist, die aus Benzophenonen, Thioxanthonen, Michlers Ketonen, Anthrachinon und Kombinationen davon besteht.

10. Photopolymerisierbare Zusammensetzung gemäß einem der Ansprüche 5 bis 9, weiterhin umfassend ein Additiv, das aus der Gruppe ausgewählt ist, die aus Pigmenten, Amin-Synergisten und Kombinationen davon besteht.

11. Photopolymerisierbare Zusammensetzung gemäß einem der Ansprüche 5 bis 10, wobei der Gehalt an dem ersten Photoinitiator im Bereich von 0,1 Gew.-% bis 15 Gew.-% liegt, bezogen auf das Gesamtgewicht der photopolymerisierbaren Zusammensetzung.

## Revendications

1. Composé photoinitiateur liquide, représenté par la formule I suivante : 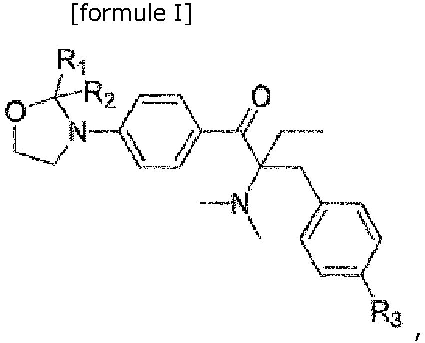 où, dans la formule I, R₁ et R₂ sont indépendamment H ou alkyle en C₁-C₃, et R₃ est H ou méthyle.

2. Composé selon la revendication 1, dans lequel R₁ et R₂ sont indépendamment méthyle, éthyle, n-propyle, ou isopropyle.

3. Composé selon la revendication 2, représenté par la formule la suivante :

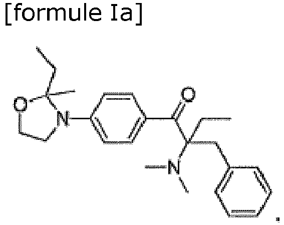

4. Procédé pour amorcer une réaction de polymérisation en utilisant le composé photoinitiateur liquide selon l'une quelconque des revendications 1 à 3.

5. Composition photopolymérisable, comprenant :
un premier photoinitiateur, qui est le composé photoinitiateur liquide selon l'une quelconque des revendications 1 à 3,
un composant photopolymérisable, et
un solvant optionnel.

6. Composition photopolymérisable selon la revendication 5, dans laquelle ledit composant photopolymérisable est un monomère oléfiniquement insaturé, un oligomère oléfiniquement insaturé, ou une combinaison de ceux-ci.

7. Composition photopolymérisable selon la revendication 6, dans laquelle ledit composant photopolymérisable est un monomère à base d'acrylate, un oligomère à base d'acrylate, ou une combinaison de ceux-ci.

8. Composition photopolymérisable selon l'une quelconque des revendications 5 à 7, comprenant en outre un second photoinitiateur choisi dans le groupe consistant en oxydes d'acylphosphine, 9,10-dialkyloxyanthracène ( 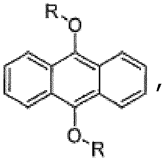 où R est alkyle), ou des combinaisons de ceux-ci.

9. Composition photopolymérisable selon l'une quelconque des revendications 5 à 8, comprenant en outre un photosensibilisateur choisi dans le groupe consistant en benzophénones, thioxanthones, des cétones de Michler, l'anthraquinone, et des combinaisons de ceux-ci.

10. Composition photopolymérisable selon l'une quelconque des revendications 5 à 9, comprenant en outre un additif choisi dans le groupe consistant en pigments, synergistes d'amines, et des combinaisons de ceux-ci.

11. Composition photopolymérisable selon l'une quelconque des revendications 5 à 10, dans laquelle la teneur du premier photoinitiateur est comprise entre 0,1 % en poids à 15 % en poids, par rapport au poids total de la composition photopolymérisable.
